# EUROPEAN PATENT APPLICATION

(11) **EP 4 799 551 A1**
(43) Date of publication of application: **02.09.2026**
(21) Application number: 25223546.0
(22) Date of filing: 15.12.2025
(51) Int. Cl.: A61B 3/00, A61B 3/135, A61B 3/14, A61B 3/12

(54) **OPHTHALMIC EQUIPMENT IMAGING SYSTEM AND OPHTHALMIC EQUIPMENT IMAGING PROGRAM**

(30) Priority: 27.02.2025 JP 2025029766
(71) Applicant: Takagi Seiko Co., Ltd., Nakano-shi, Nagano 383-8585 (JP)
(72) Inventor: NAGAI, Kazuki, Nakano-shi, 383-8585 (JP)
(74) Representative: Mewburn Ellis LLP

(57) **Abstract**

A target position for calculating exposure time is automatically calculated and an appropriate exposure time is easily calculated. A system (10) to do this includes: a camera (22) that performs image capture of an image capture target range including a subject's eye to be observed by ophthalmic equipment (20); and exposure time calculation means (24) for calculating an exposure time of the camera (22). The exposure time calculation means (24) divides the image capture target range into areas of different brightness and calculates the exposure time in keeping with a brightness of an area specified by an operator.

## Description

### Technical Field

The present invention relates to an imaging system and an imaging program for image capture of a subject's eye.

### Background Art

One example of ophthalmic equipment is a slit lamp microscope. A slit lamp microscope is an ophthalmic instrument that illuminates a subject's eye from an oblique angle with slit light to make transparent or translucent tissue visible and thereby enable observation of the subject's eye (such as the anterior segment).

As one example, Patent Document 1 (Japanese Patent No. 3118862) discloses a slit lamp microscope equipped with a slit lamp illumination unit that emits slit light, a microscope unit with an eyepiece for observing the subject's eye, and a camera disposed at a branch on an optical path from the subject's eye to the microscope unit.

Patent Document 1 also uses a method in which predetermined combinations of an image capture position in the subject's eye and an image capture method are presented to the operator, an operation whereby the operator selects one of the combinations is received, coefficients for the combinations of the image capture location and the image capture method are stored in advance, and the exposure is calculated using the coefficients and a predetermined formula.

### Summary of Invention

### Technical Problem

Since a slit lamp microscope is used in a dark environment where there is little peripheral light and only the area illuminated with slit light is bright, it is extremely difficult for a camera to capture a clear image. In other words, since only the part illuminated with slit light is bright and other parts are dark, it is typical for bright areas in the image to be washed out and for dark areas to become crushed.

Although the configuration disclosed in Patent Document 1 is designed to calculate the appropriate exposure, there is the problem that combinations of the image capture position and the image capture method need to be set in advance, making this technology unsuited to general use.

The present invention was conceived to solve the above problem and has an object of providing an ophthalmic equipment imaging system and an ophthalmic equipment imaging program that can automatically calculate a target position for calculating the exposure time and can set an appropriate exposure time.

### Solution to Problem

An ophthalmic equipment imaging system according to an aspect of the present disclosure includes: a camera that performs image capture of an image capture target range including a subject's eye to be observed by ophthalmic equipment; and exposure time calculation device for calculating an exposure time of the camera, wherein the exposure time calculation device divides the image capture target range into areas of different brightness and calculates the exposure time in keeping with a brightness of an area specified by an operator. According to this configuration, since the image capture target range is divided into areas based on brightness and the exposure time is calculated based on the brightness of a specified area, it is possible to easily calculate an appropriate exposure time.

In the ophthalmologic equipment imaging system described above, the exposure time calculation device creates a brightness value-converted image by converting a live view image of the image capture target range based on brightness values, sets a predetermined range in the brightness value-converted image as an exposure time calculation range, divides the exposure time calculation range into an area whose brightness is equal to or greater than a preset threshold and an area whose brightness is less than the threshold, and calculates, based on an exposure time measurement algorithm set in advance, the exposure time for the area specified by the operator out of the area whose brightness is equal to or greater than the threshold and the area whose brightness is less than the threshold. With this configuration, an exposure time based on an area with high brightness or an exposure time based on an area with low brightness can be easily calculated.

The system may further include HDR processing device for acquiring the image capture target range as a high brightness image and a low brightness image and creating an HDR-processed image of the image capture target range. The image captured by the camera can be made easy to view and similar to an actual scene viewed by a human.

### Advantageous Effects of Invention

According to the present invention, a target position for calculating exposure time can be automatically calculated and an appropriate exposure time can be easily calculated.

### Brief Description of Drawings

Fig. 1 is a diagram useful in explaining the overall configuration of an ophthalmic equipment imaging system.
Fig. 2 is a side view of a slit lamp microscope.
Fig. 3 is a block diagram depicting the internal configuration of a computer.
Fig. 4 is a flowchart useful in explaining an exposure time calculation operation.
Fig. 5 is a diagram useful in explaining one example of a live view image.
Fig. 6 is a diagram useful in explaining one example of a brightness value-converted image.
Fig. 7 is a diagram useful in explaining one example where an exposure time calculation range has been divided into an area whose brightness is equal to or greater than a threshold and an area whose brightness is less than the threshold.
Fig. 8 is a diagram useful in explaining another example where an exposure time calculation range has been divided into an area whose brightness is equal to or greater than a threshold and an area whose brightness is less than the threshold.

### Description of Embodiments

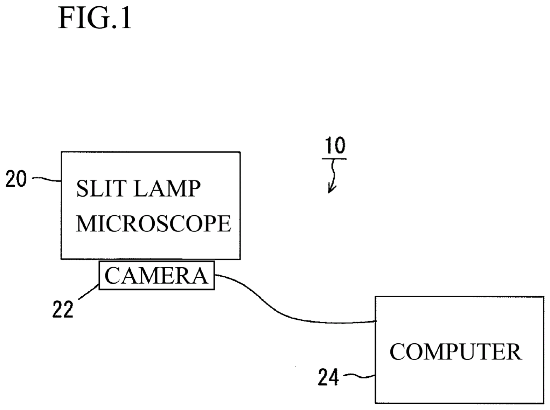

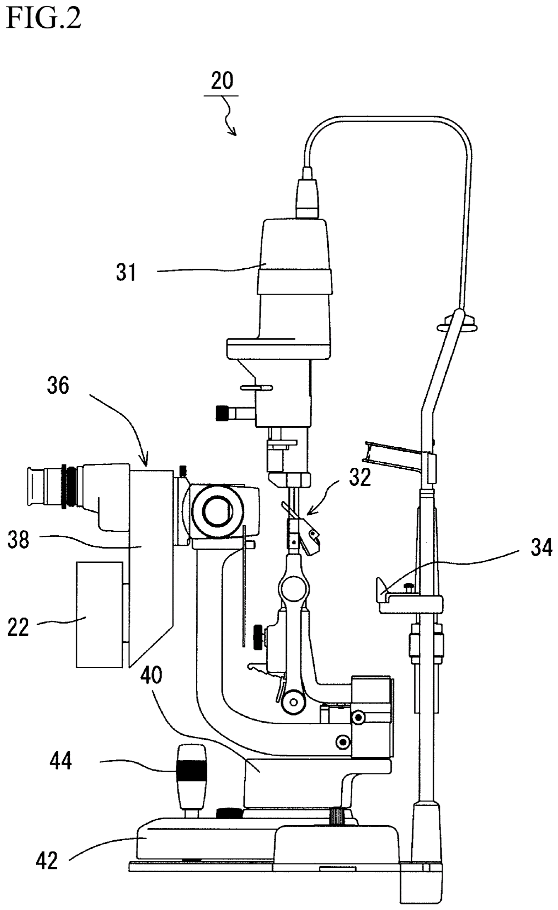

Figs. 1 and 2 depict the overall configuration of an ophthalmic equipment imaging system according to the present embodiment. Note that in the following explanation, a slit lamp microscope is described as one example of ophthalmic equipment.

An ophthalmic equipment imaging system 10 according to the present embodiment includes a camera 22 for image capture of the subject's eye and a computer 24. The camera 22 is provided on a slit lamp microscope 20. The camera 22 and the computer 24 are connected for the purpose of data communication, and this may be a wired or wireless connection.

The slit lamp microscope 20 is provided with a slit light unit 31 that emits slit light, a mirror unit 32 that reflects the emitted slit light toward the subject's eye, and a chin rest 34 on which the subject rests their chin. A microscope 36 for observing the eye of a subject who has placed their chin on the chin rest 34 is provided in a position facing the chin rest 34.

The microscope 36 has a camera mounting unit 38 that has an internal beam splitter (not illustrated) and enables the camera 22 to be mounted. The camera mounting unit 38 is located closer to the subject's eye than the eyepiece 39, and splits light from the subject's eye between the eyepiece 39 side and the camera 22 side.

The microscope 36, the slit light unit 31, and the mirror unit 32 are attached to a fine adjustment base 40. The fine adjustment base 40 is provided so as to enable fine adjustment on the horizontal plane relative to a base 42. Fine adjustment of the fine adjustment base 40 can be performed by operating a joystick 44.

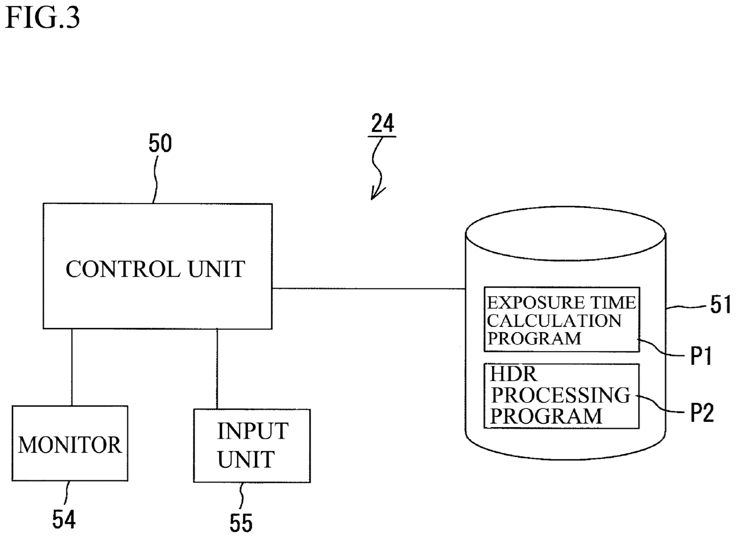

Fig. 3 is a block diagram depicting the internal configuration of the computer 24 connected to the camera 22. A typical personal computer can be used as the computer 24. The computer 24 includes a control unit 50 including a CPU, memory, and the like, a storage unit 51 including a hard disk drive, an SSD, or the like, a monitor 54, and an input unit 55 including a mouse, keyboard, or the like. The computer 24 is operated by an operator, such as an ophthalmologist.

The storage unit 51 stores an exposure time calculation program P1, which calculates an appropriate exposure time based on a live view image in the camera 22. The control unit 50 realizes an exposure time calculation device for calculating an appropriate exposure time by reading and executing the exposure time calculation program P1.

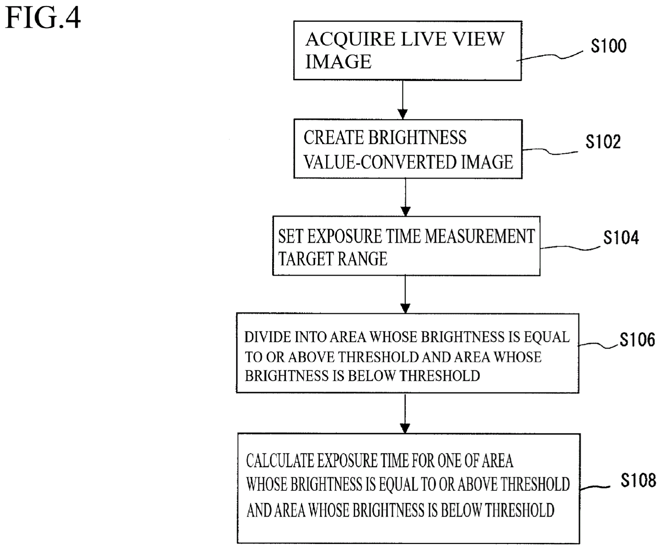

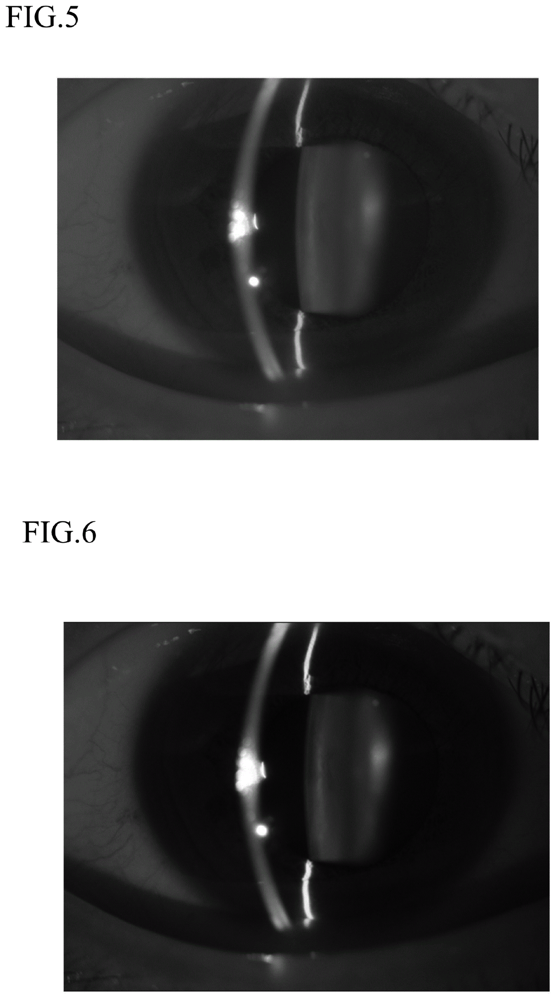

Fig. 4 is a flowchart of an exposure time calculation operation. When the control unit 50 executes the exposure time calculation program P1, a live view image at the camera 22 is acquired (step S100). As depicted in Fig. 5, two types of light can be seen in this live view image, that is, slit light that is relatively wide and has been reflected from the surface of the subject's eye, and slit light that is relatively narrow and has been reflected from the interior of the subject's eye. The relatively narrow slit light reflected from the interior of the subject's eye is not reflected at the pupil and can be seen on both sides of the pupil.

The control unit 50 creates a brightness value-converted image by converting the acquired live view image based on brightness values (step S102). A brightness value-converted image is depicted in Fig. 6, in which the positions with high brightness appear as white. Positions with the relatively wide slit light reflected at the surface of the subject's eye and the areas with the relatively narrow slit light reflected from the interior of the subject's eye correspond to such positions with high brightness.

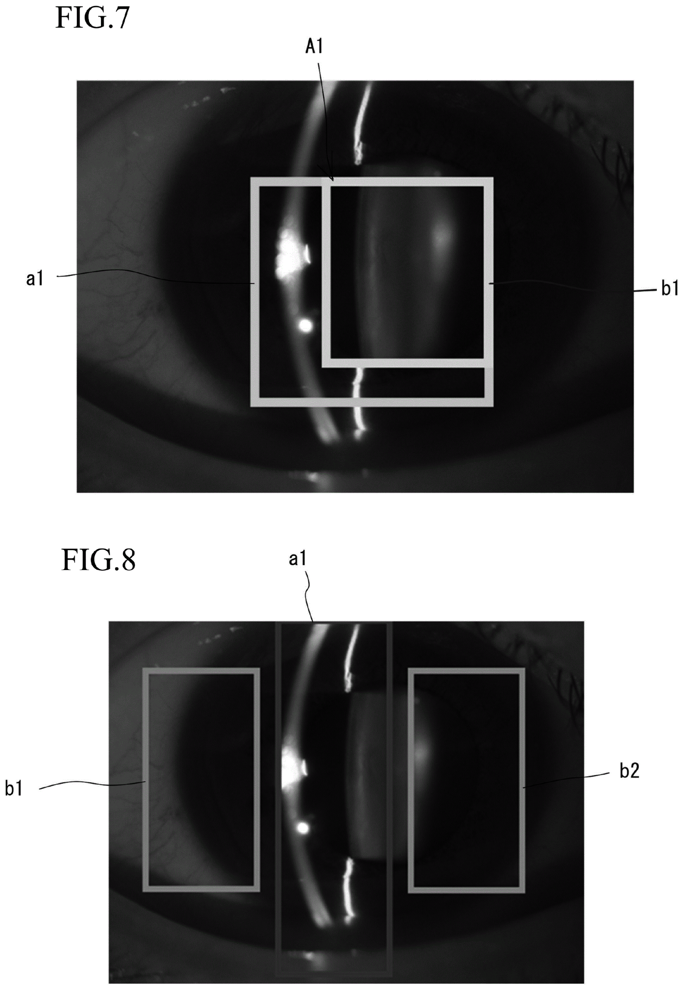

The control unit 50 sets a predetermined position in the brightness value-converted image as an exposure time measurement target range (step S104). As one example, as depicted in Fig. 7, it is possible to set 50% of the total area in the center of an image capture target range as the exposure time measurement target range. In Fig. 7, a square area A1 positioned in the center of the brightness value-converted image is set as the exposure time measurement target range. However, as the exposure time measurement target range, it is possible for the operator to freely change where the location of the image capture target range is to be set and what percentage of the area of the image capture target range is to be used.

The control unit 50 divides the exposure time calculation range A1 into an area a1 whose brightness is equal to or greater than a preset threshold and an area b1 whose brightness is less than the threshold (step S106). In Fig. 7, the area a1 whose brightness is equal to or greater than the threshold is an L-shaped area that extends from the left and along the bottom of exposure time calculation range A1, and the area b1 whose brightness is less than the threshold is a rectangular area on the right side of the exposure time calculation range A1. However, it is assumed that the brightness threshold is also freely changeable as desired by the operator.

Based on an instruction from the operator, the control unit 50 calculates the exposure time for either the area a1 whose brightness is equal to or greater than the threshold or the area b1 whose brightness is less than the threshold based on a preset exposure time measurement algorithm (step S108). Note that a typical calculation formula can be used as the exposure time measurement algorithm.

Note that the area for which the exposure time is to be calculated out of the area a1 whose brightness is equal to or greater than the threshold and the area b1 whose brightness is less than the threshold can be set by the operator operating the input unit 55 to indicate the area for which the exposure time is to be calculated every time calculation is performed, or by the operator setting the area for which the exposure time is to be calculated in advance as an initial setting. It is assumed that the area for which the exposure time is to be calculated in the initial setting is also changeable.

The control unit 50 displays the exposure time calculated based on the selected area on the monitor 54. The operator views the exposure time displayed on the monitor 54 and operates the camera 22 accordingly.

Note that in step S106, there may be a plurality of areas whose brightness is equal to or greater than the threshold or areas whose brightness is less than the threshold. In the example depicted in Fig. 8, the area a1 whose brightness is equal to or greater than the threshold includes the area with the relatively wide slit light reflected at the surface of the subject's eye and the area with the relatively narrow slit light reflected at the interior of the subject's eye, and the left and right sides of this area are set as areas b1 and b2 whose brightness is less than the threshold.

As described above, since the exposure time calculation range is divided into an area whose brightness is equal to or greater than the threshold and an area whose brightness is less than the threshold and the exposure time is calculated based on whichever of the areas the operator wishes to observe, it is possible for the operator to obtain a clear image of the area the operator wishes to observe.

The storage unit 51 may also store an HDR processing program P2 that acquires the image capture range as a bright image and a dark image and creates an HDR-processed image of the image capture range. The control unit 50 realizes an HDR processing device for creating an HDR-processed image by reading and executing the HDR processing program P2.

The expression "HDR processing" refers to High Dynamic Range processing, which, through well-known processing, makes it possible to combine bright images and dark images to produce an easy-to-view image that is similar to an actual scene viewed by a human.

Although a configuration where the exposure time calculation device is executed by a computer provided separately from the camera 22 has been described in the embodiment given above, the exposure time calculation device may be provided in the camera 22 itself.

The ophthalmic equipment mentioned here is not limited to a slit lamp microscope and
the present invention can be applied to other equipment used to observe a subject's eye, such as a surgical microscope, a contrast glare tester, and a fundus examination device.

## Claims

1. An ophthalmic equipment imaging system (10) comprising:
a camera (22) that performs image capture of an image capture target range including a subject's eye to be observed by ophthalmic equipment (20); and
exposure time calculation means (24) for calculating an exposure time of the camera (22),
wherein the exposure time calculation means (24) divides the image capture target range into areas of different brightness and calculates the exposure time in keeping with a brightness of an area specified by an operator.

2. The ophthalmologic equipment imaging system (10) according to claim 1,
wherein the exposure time calculation means (24) creates a brightness value-converted image by converting a live view image of the image capture target range based on brightness values, sets a predetermined range in the brightness value-converted image as an exposure time calculation range, divides the exposure time calculation range into an area whose brightness is equal to or greater than a preset threshold and an area whose brightness is less than the threshold, and calculates, based on an exposure time measurement algorithm set in advance, the exposure time for the area specified by the operator out of the area whose brightness is equal to or greater than the threshold and the area whose brightness is less than the threshold.

3. The ophthalmic equipment imaging system (10) according to claim 1 or 2,
further comprising HDR processing means (50) for acquiring the image capture target range as a high brightness image and a low brightness image and creating an HDR-processed image of the image capture target range.

4. An ophthalmic equipment imaging program that is readable by a computer (24) connected to a camera (22) that performs image capture of an image capture target range including a subject's eye to be observed by ophthalmic equipment (20), the ophthalmic equipment imaging program comprising causing the computer (24) to realize:
a function of dividing the image capture target range into areas of different brightness;
a function of urging an operator to input a specified area; and
a function of calculating an exposure time in keeping with a brightness of the area specified by the operator.

5. The ophthalmic equipment imaging program according to claim 4,
further comprising causing the computer (24) to realize:
a function of creating a brightness value-converted image by converting a live view image of the image capture target range based on brightness values;
a function of setting a predetermined range in the brightness value-converted image as an exposure time calculation range;
a function of dividing the exposure time calculation range into an area whose brightness is equal to or greater than a preset threshold and an area whose brightness is less than the threshold; and
a function of calculating, based on an exposure time measurement algorithm set in advance, the exposure time for the area specified by the operator out of the area whose brightness is equal to or greater than the threshold and the area whose brightness is less than the threshold.

6. The ophthalmic equipment imaging program according to claim 4 or 5,
further comprising causing the computer (24) to realize an HDR processing function that acquires the image capture target range as a high brightness image and a low brightness image and creates an HDR-processed image of the image capture target range.
